# DEMANDE DE BREVET EUROPEEN

(11) **EP 0 676 200 A2**
(43) Date de publication de la demande: **11.10.1995**
(21) Numéro de dépôt: 95400433.9
(22) Date de dépôt: 01.03.1995
(51) Int. Cl.: A61K 31/445

(54) **Utilisation de l'éliprodil et de ses énantiomères pour la préparation de médicaments utiles dans le traitement des neuropathies périphériques et des maladies neurodégénératives centrales**

(30) Priorité: 09.03.1994 FR 9402699
(71) Demandeur: SYNTHELABO, F-92350 Le Plessis Robinson (FR)
(72) Inventeur: Poindron, Philippe, B.P. 24 F-67401 Illkirch Cedex (FR); Braun, Serge, B.P. 24 F-67401 Illkirch Cedex (FR); Ferzaz, Badia, F-92160 Antony (FR)
(74) Mandataire: Thouret-Lemaitre, Elisabeth

(57) **Abrégé**

Utilisation de l'éliprodil et de ses énantiomères pour la préparation de médicaments utiles dans le traitement des neuropathies périphériques et des maladies neurodégénératives centrales.

## Description

La présente invention a pour objet l'utilisation de l'éliprodil et de ses énantiomères pour la préparation de médicaments utiles dans le traitement des neuropathies périphériques et des maladies neurodégénératives centrales.

L'éliprodil, composé de formule (I) 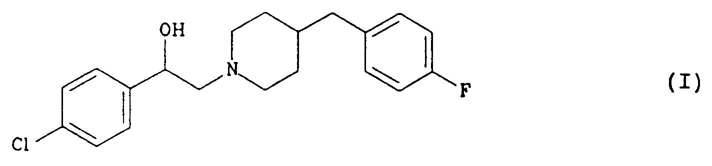
est un composé connu dont les propriétés neuroprotectrices ont fait l'objet du brevet européen n° 0 109 317.

Ce composé possède également une activité antipsychotique qui a fait l'objet de la demande de brevet FR 89 14868.

L'éliprodil et ses énantiomères ont été soumis à de nouveaux essais pharmacologiques qui ont mis en évidence leurs propriétés neurotrophes.
En particulier, les effets neuritogène et synaptogène de l'éliprodil et de ses énantiomères ont été déterminés in vitro dans un modèle de culture mixte de fibres musculaires humaines et de neurones de moelle épinière de rat.
Cet essai a été réalisé de la façon suivante :

Des fragments de muscle, débarrassés de leur gaine fibreuse et découpés en petits fragments sont laissés une nuit dans un milieu de conditionnement composé de milieu 199 à 10% de sérum de veau foetal (SVF) et 1% de solution antibiotique et antifongique prête à l'emploi [benzylpénicillinate de sodium, streptomycine, fungizone (GIBCO)]. Ces fragments sont ensuite maintenus pendant 5 à 8 jours dans un coagulum nutritif constitué de 4 volumes de milieu de conditionnement pour 1 volume de plasma humain.

Les explants sont ensuite transférés dans des boîtes de Pétri gélatinées et sont humidifiés et immobilisés sur le support par incubation pendant 1 h à 37°C. On ajoute alors du milieu F14 (GIBCO) contenant 10 % de SVF, de la glutamine (2 mM), de l'insuline (10 µg/ml), du FGF (10 ng/ml) et de l'EGF (10 ng/ml).
Une abondante population de cellules musculaires satellites (précurseurs chez l'adulte des fibres musculaires) migre hors de l'explant. Ces cellules prolifèrent et commencent à fusionner après une semaine de culture environ.
Les explants sont retirés avant fusion des cellules satellites en myotubes.
Les boîtes sont traitées par de la trypsine juste avant la phase de fusion et les cellules sont subcultivées afin d'obtenir la quantité nécessaire aux expériences.

Les cellules sont finalement ensemencées à raison de 20 000 cellules par cm². Après formation des myotubes, on dépose des explants de moelle épinière d'embryon de rat (13ème jour de gestation) qui sont immobilisés sur la couche de cellules musculaires. Le milieu utilisé pour la co-culture est constitué de 25 % de milieu 199, de 67,5 % de milieu MEM (GIBCO), de 5 % de SVF, d'insuline (10 µg/ml) et de solution antibiotique (1 %). Le milieu est renouvelé 2 fois par semaine. Les produits à étudier sont mis en solution dans ce milieu de culture.

Dans la technique de co-culture utilisée, tous les explants de moelle épinière émettent des neurites mais tous ne parviennent pas à innerver les cellules musculaires et à induire leur contraction. Cette contraction est l'indice d'une innervation efficace des fibres musculaires. Dans des conditions standard, 1 explant sur 4 parvient à établir des contacts fonctionnels avec ces fibres. Ces conditions sont très favorables à la mise en évidence d'une activité promotrice de la synaptogénèse.
L'effet des produits, déterminé après 3-4 semaines de traitement, a été évalué par comparaison avec le véhicule ayant servi à leur solubilisation (éthanol à 0,1 % pour la concentration de 10⁻⁶ M).
Cet effet a été déterminé à des concentrations de 10⁻¹¹, 10⁻⁹, 10⁻⁷ et 10⁻⁶ M des produits.
On a mesuré les paramètres suivants :
1) Longueur des neurites
   La longueur des neurites est déterminée par examen des cultures au microscope à contraste de phase muni d'un micromètre oculaire à un grossissement final de x 200. On mesure la longueur des neurites à partir du centre de l'explant et sans tenir compte de la courbure éventuelle de ces extensions filamenteuses. Les branchements sont également mesurés.
   On détermine la longueur totale moyenne des neurites par explant. La mesure est faite sur au moins 15 explants.
2) Nombre de neurites par explant
   Le dénombrement des neurites se fait en comptant les fibres émergeant de l'explant sans prise en compte des embranchements.
3) Nombre de jonctions neuromusculaires.
   - Dénombrement des agrégats de récepteurs cholinergiques.
      Les cultures sont incubées pendant 1 h en présence d'α-bungarotoxine marquée à l'iode 125. Elles sont ensuite fixées par une solution de glutaraldéhyde à 2,5 %, séchées et mises en contact avec une émulsion photographique fluide. Les autoradiogrammes sont développés, après 10 jours d'exposition. Après développement, les cultures sont observées au microscope (grossissement x 200). On choisit des fibres musculaires bien isolées dont les agrégats sont bien séparés les uns des autres. Ces fibres ont en général une longueur très supérieure au diamètre du champ et c'est donc ce diamètre qui sert d'unité de longueur. Au moins soixante fibres sont étudiées. La valeur choisie est la moyenne des nombres d'agrégats, multipliée par un facteur de correction qui ramène au mm.
   - Dénombrement des plages d'acétylcholinestérase.
      L'acétylcholinestérase colocalise avec les agrégats de récepteurs cholinergiques. Elle est révélée par la méthode de Karnovsky et Roots, modifiée par Kobayashi et Askanas (J. Neurosci. vol 7, 3131-3141, 1987). Les plages sont dénombrées en suivant les mêmes principes que pour le dénombrement des agrégats de récepteurs et sont quantitativement et topologiquement équivalentes.
4) Surface des zones innervées.
   - surface totale des zones de cellules musculaires innervées autour de l'explant.
      Cette valeur rend compte de la couverture motoneuronale sans préjuger de la présence d'espaces et/ou d'autres types cellulaires.
   - surface réelle occupée par les fibres musculaires innervées
      Ces zones sont révélées soit par autoradiographie des agrégats de récepteurs cholinergiques, soit par coloration des plages d'acétylcholinestérase.
      Cette valeur rend compte de la quantité de fibres musculaires innervées.
      Ces surfaces sont déterminées après digitalisation et analyse d'image.

On a trouvé que l'éliprodil et ses énantiomères accentuent la croissance neuritique par rapport aux témoins à partir de la concentration de 10⁻⁷ M dans les explants donnant lieu ou pas à innervation. A la concentration de 10⁻⁷ M d'éliprodil, la longueur des neurites augmente en moyenne de respectivement 25 et 28% dans les explants donnant lieu ou pas à innervation.
L'éliprodil augmente également le nombre de neurites. A la concentration de 10⁻⁶ M, cette augmentation est en moyenne de 156% dans les explants donnant lieu à innervation et de 100% dans les explants ne donnant pas lieu à innervation. Ce composé augmente également le nombre de jonctions neuromusculaires formées et l'étendue des zones de fibres musculaires innervées à partir d'une concentration de 10⁻⁷ M. A la concentration de 10⁻⁶ M, le nombre de jonctions neuromusculaires croît en moyenne de 60 %, la surface totale des zones de cellules musculaires innervées de 116 % et la surface réelle des fibres musculaires innervées de 142 %.

De la même façon, à la concentration de 10⁻⁷ M, les énantiomères S(+) et R(-) augmentent de respectivement 68 % et 32 % la longueur des neurites dans les zones innervées et de respectivement 126 % et 162 % le nombre de neurites dans les explants donnant lieu à innervation.

L'effet de l'éliprodil sur la régénération du nerf sciatique in vivo a également été étudié.

Cet effet a été évalué après lésion par congélation locale du nerf sciatique chez le rat.

La lésion par congélation détruit les fibres du nerf sciatique qui subissent une dégénérescence wallérienne au site de la lésion et dans tout le tronçon distal. Ce type de lésion conserve les gaines nerveuses et permet une régénération nerveuse dans des conditions reproductibles. Le processus de régénération commence à partir du côté proximal dans les heures qui suivent la lésion. La vitesse de régénération des fibres sensitives est mesurée par un test de pincement (pinch-test) 8 jours après la lésion.

Les animaux sont des rats adultes mâles de souche Sprague Dawley (Iffa Credo) pesant 250 g environ. Après anesthésie des animaux par du pentobarbital sodique (60 mg/kg), la peau de la cuisse est désinfectée à l'alcool et incisée au niveau de la jonction des biceps fémoraux. Le nerf sciatique est mis à nu après avoir écarté les muscles Lateralis et le Biceps Femoris. Le point de la lésion est repéré par une microsuture (Ethilon noir 10-0) sur le périnèvre au dessus de la trifurcation du nerf sciatique. La lésion du nerf sciatique est effectuée sur 1 mm par 6 cycles de congélation-décongélation à l'aide d'une cryode en cuivre pré-refroidie dans de l'azote liquide. La plaie est ensuite refermée et traitée par un antibiotique (Exoseptoplix®). Les animaux sont mis en cages individuelles et surveillés quotidiennement.

Après l'opération, les animaux sont répartis en 4 lots de 6 individus :
- témoins lésés qui reçoivent une injection i.p. de tween 80 à 0,1 %, deux fois par jour,
- témoins lésés qui reçoivent une injection i.p. de tween une fois tous les 3 jours,
- animaux lésés traités deux fois par jour par l'éliprodil à 1 mg/kg ip,
- animaux lésés injectés tous les 3 jours par l'éliprodil à 10 mg/kg ip.

Huit jours après l'opération, les animaux sont légèrement anesthésiés, et le nerf sciatique est remis à nu pour effectuer le pinch-test. Ce test consiste à pincer légèrement le nerf à l'aide d'un forceps en commençant par la région la plus distale du nerf et en remontant tous les 0,5 mm. Une réponse reflexe (contraction des muscles de la région postérieure de l'animal) est observée là où le front des fibres sensitives régénérées est présent. Ce point est marqué par une microsuture. Le nerf est ensuite prélevé, la distance entre le site de la lésion et la microsuture distale est mesurée sur un papier millimétré sous microscope opératoire. Après prélèvement du nerf les animaux sont sacrifiés par une overdose de pentobarbital.

Chez les témoins lésés, le pinch-test effectué 8 jours après la lésion a montré une réaction des animaux à 25 mm en moyenne; cette longueur correspond à la distance parcourue par les fibres sensitives régénérées en 8 jours. Les 2 lots de témoins injectés quotidiennement et tous les 3 jours ont donné des résultats identiques. Chez les animaux traités à l'éliprodil, l'injection deux fois par jour de 1 mg/kg augmente la distance parcourue par les fibres sensitives de 14 %. Le traitement à la dose de 10 mg/kg tous les 3 jours augmente la distance parcourue par les fibres sensitives de 10 %.
Ces résultats indiquent que l'éliprodil stimule la régénération du nerf sciatique lésé in vivo.

Les résultats des essais pharmacologiques réalisés in vitro et in vivo ont montré que l'éliprodil et ses énantiomères favorisent la croissance, la réparation et la régénération de l'axone des neurones et la formation des jonctions neuromusculaires. Ils sont donc utilisables dans le traitement des neuropathies périphériques, telles que neuropathies traumatiques (section ou écrasement d'un nerf) ou ischémiques, neuropathies métaboliques (diabète, urémie), neuropathies infectieuses, alcooliques et médicamenteuses, neuropathies génétiques, etc..., dans les affections du motoneurone telles que les amyotrophies spinales et la sclérose latérale amyotrophique, et dans le traitement des maladies neurodégénératives chroniques impliquant une dégénerescence axonale cérébrale (maladie d'Alzheimer, maladie de Parkinson, etc...) et de la sclérose en plaque.

L'éliprodil et ses énantiomères peuvent être présentés sous toutes formes galéniques, en association avec des excipients appropriés, pour une administration orale, parentérale ou locale, par exemple sous forme de comprimés, gélules, solutions, patch, dosés pour permettre l'administration d'une dose journalière de 1 à 30 mg de principe actif.

## Revendications

1. Utilisation de l'éliprodil et de ses énantiomères, sous forme de base libre ou de sel d'addition à un acide, pour la préparation de médicaments utiles dans le traitement des neuropathies périphériques et des maladies neurodégénératives centrales.
